# EUROPEAN PATENT APPLICATION

(11) **EP 2 052 720 A2**
(43) Date of publication of application: **29.04.2009**
(21) Application number: 09150175.9
(22) Date of filing: 22.11.2004
(51) Int. Cl.: A61K 31/13, A61K 31/445, A61K 31/42, A61P 27/02, A61P 27/06, A61P 43/00

(54) **Use of at least one hydroxylamine compound for the treatment of eye disease**

(30) Priority: 20.11.2003 US 523802 P
(62) Divisional of application: 04812278.2
(71) Applicant: Othera Holding, Inc., Wilmington, DE 19805 (US)
(72) Inventor: Matier, William, L, Hockessin, DE 19707 (US); Patil, Ghanshyam, Lincoln University, PA 19352 (US)
(74) Representative: Portal, Gérard

(57) **Abstract**

Uses for the treatment or prevention of a number of ocular diseases or disorders are disclosed. In particular, uses are disclosed for the amelioration of diabetic retinophaty and other retinopathies, as well as uveitis, presbyopia, dry eye, glaucoma, blepharitis and rosacea of the eye. The uses comprise administration of a composition comprising an ophtalmalogically acceptable carrier or diluent and a hydroxylamine compound in a therapeutically sufficient amount to prevent, retard the development or reduce the symptoms of one or more of the ophthalmic conditions.

## Description

### FIELD OF THE INVENTION

The present invention relates to the treatment of ophthalmic diseases. More specifically, the invention provides uses for reducing or preventing deleterious conditions of the retina and surrounding tissues, through the administration of pharmaceutical preparations comprising hydroxylamine compounds.

### BACKGROUND OF THE INVENTION

Various patents and other publications are referenced herein. The contents of each of these patents and publications are incorporated by reference herein, in their entireties.

As a complex and sensitive organ of the body, the eye can experience numerous diseases and other deleterious conditions that affect its ability to function normally. Many such conditions can be found in the interior and most particularly at the rear of the eye, where lie the optic nerve and the retina, seven layers of alternating cells and processes that convert a light signal into a neural signal. Diseases and degenerative conditions of the optic nerve and retina are the leading causes of blindness throughout the world.

A significant degenerative condition of the retina is macular degeneration, also referred to as age-related macular degeneration (AMD). AMD is the most common cause of vision loss in the United States in those 50 or older, and its prevalence increases with age. AMD is classified as either wet (neovascular) or dry (non-neovascular). The dry form of the disease is most common. It occurs the central retina has become distorted, pigmented, or most commonly, thinned. The wet form of the disease is responsible for most severe loss of vision. The wet form of macular degeneration is usually associated with aging, but other diseases that can cause wet macular degeneration include severe myopia and intraocular infections like histoplasmosis, which may be exacerbated in individuals with AIDS. A variety of elements may contribute to macular degeneration, including genetic makeup, age, nutrition, smoking and exposure to sunlight.

Retinopathy associated with diabetes is a leading cause of blindness in type 1 diabetes, and is also common in type 2 diabetes. The degree of retinopathy depends on the duration of the diabetes, and generally begins to occur ten or more years after onset of diabetes. Diabetic retinopathy may be classified as (1) non-proliferative or background retinopathy, characterized by increased capillary permeability, edema, hemorrhage, microaneurysms, and exudates, or (2) proliferative retinopathy, characterized by neovascularization extending from the retina to the vitreous, scarring, fibrous tissue formation, and potential for retinal detachment. Diabetic retinopathy is believed to be caused, at least in part, by the development of glycosylated proteins due to high blood glucose. Glycosylated proteins generate free radicals, resulting in oxidative tissue damage and depletion of cellular reactive oxygen species (ROS) scavengers, such as glutathione.

Several other less common, but nonetheless debilitating retinopathies include choroidal neovascular membrane (CNVM), cystoid macular edema (CME, also referred to as macular edema, macular swelling), epi-retinal membrane (ERM) (macular pucker) and macular hole. In CNVM, abnormal blood vessels stemming from the choroid grow up through the retinal layers. The fragile new vessels break easily, causing blood and fluid to pool within the layers of the retina. In CME, which can occur as a result of disease, injury or surgery, fluid collects within the layers of the macula, causing blurred, distorted central vision. ERM (macular pucker) is a cellophane-like membrane that forms over the macula, affecting the central vision by causing blur and distortion. As it progresses, the traction of the membrane on the macula may cause swelling. ERM is seen most often in people over 75 years of age. Its etiology is unknown, but may be associated with diabetic retinopathy, posterior vitreous detachment, retinal detachment or trauma, among other conditions.

Another disease of the interior of the eye is uveitis, or inflammation of the uveal tract. The uveal tract (uvea) is composed of the iris, ciliary body, and choroid. The uvea is the intermediate of the three coasts of the eyeball, between the sclera the retina in its posterior (choroid) portion. Uveitis may be caused by trauma, infection or surgery, and can affect any age group. Uveitis is classified anatomically as anterior, intermediate, posterior, or diffuse. Anterior uveitis affects the anterior portion of the eye, including the iris. Intermediate uveitis, also called peripheral uveitis, is centered in the area immediately behind the iris and lens in the region of the ciliary body. Posterior uveitis may also constitute a form of retinitis, or it may affect the choroids or the optic nerve. Diffuse uveitis involves all parts of the eye.

Glaucoma is made up of a collection of eye diseases that cause vision loss by damage to the optic nerve. Elevated intraocular pressure (IOP) due to inadequate ocular drainage is a primary cause of glaucoma. Glaucoma can develop as the eye ages, or it can occur as the result of an eye injury, inflammation, tumor or in advanced cases of cataract or diabetes. It can also be caused by certain drugs such as steroids. Further, glaucoma can develop in the absence of elevated IOP. This form of glaucoma has been associated with inheritance (i.e., family history of normal-tension glaucoma) Japanese ancestry, as well as systemic heart disease, such as irregular heartbeat.

The eye produces about one teaspoon of aqueous humor daily. Normally, this fluid escapes from the eye through a spongy mesh of connective tissue called the trabecular meshwork at the same rate at which it is produced. Free radicals and other reactive oxygen species (ROS) cause gradual damage to the trabecular meshwork over a period of time. As a result, the trabecular meshwork becomes partially blocked, outflow facility decreases and the IOP builds up as more aqueous humor is formed. Though the IOP does not rise high enough to cause any noticeable symptoms initially, when pressure remains elevated or continues to rise, fibers in the optic nerve are compressed and destroyed, leading to a gradual loss of vision over a period of years. Izzotti et al. provide convincing evidence linking oxidative DNA damage in a small but critical tissue structure in the outflow system to glaucoma ( Izzotti A, Sacca SC, Cartiglia C, De Flora S. Oxidative deoxyribonucleic damage in the eyes of glaucoma patients. Am J Med. 2003; 114:638-646). They observed a more than threefold increase in the amount of 8-oxo-deoxyguanosine (8-OH-dG) in the trabecular meshwork tissue of glaucoma patients. The increased oxidative DNA damage correlated further with clinical parameters, such as intraocular pressure indexes and visual field loss.

The primary features of the optic neuropathy in glaucoma include characteristic changes in the optic nerve head, a decrease in number of surviving retinal cells, and loss of vision. It has been proposed that a of events links degeneration of the optic nerve head with the slow death of retinal ganglion cells observed in the disease, and that this cascade of events can be slowed or prevented through the use of neuroprotective agents (Osborne et al., 2003, Eur. J. Ophthalmol, 13 (Supp3): S19-S26), of which antioxidants and free radical scavengers are an important class (Hartwick, 2001, Optometry and Vision Science 78: 85-94).

A less serious but more pervasive condition of the eye is presbyopia, which affects the ocular lens. The lens is enveloped by a tough collagen capsule that is thought to impart elasticity to the lens, enabling it to focus at different distances through ciliary muscle-controlled changes of curvature. As the lens ages, it increases in volume and hence progressively loses its elasticity, which diminishes an individual's ability to focus on near objects.

The eye's outermost layer, the cornea, controls and focuses the entry of light into the eye. The cornea must remain transparent to refract light properly. The cornea also helps to shield the rest of the eye from germs, dust, and other harmful matter, and, significantly, it serves as a filter to screen out some of the most damaging ultraviolet (UV) wavelengths in sunlight. Without this protection, the lens and the retina would be highly susceptible to injury from UV radiation.

The cornea and surrounding conjunctiva are also subject to a variety of deleterious conditions that can impair vision. These include inflammatory responses, such as those resulting from allergic reaction, infection or trauma, and a variety of dystrophies (conditions in which one or more parts of the cornea lose their normal clarity due to a buildup of cloudy material), such as Fuchs' dystrophy, keratoconus, lattice dystrophy, and map-dot-fingerprint dystrophy, to name a few, as well as other disorders (*e.g.*, dry eye syndrome).

Ocular surface and lacrimal gland inflammation has been identified in dry eye that plays a role in the pathogenesis of ocular surface epithelial disease, termed keratoconjunctivitis sicca. Both oxidative tissue damage and polymorphonuclear leukocytes indicating an oxidative potential occur in the tear film of patients suffering from dry eyes. These reactions lead to severe damage of the involve tissue. Free radicals and inflammation may be involved in the pathogenesis or in the self-propagation of the disease. (Augustin, A.J. et al., "Oxidative reactions in the tear fluid of patients suffering from dry eyes" Graefe's Arch. Clin.l Exp.l Ophthalmol. 1995, 11:694-698).

Retinal phototoxicity is induced by exposure of the eye to retinal illumination from an operating microscope positioned for temporal approach eye surgery or from lasers used by the military. These light sources have the potential for light-induced injury to the fovea (M.A. Pavilack and R.D. Brod "Site of Potential Operating Microscope Light-induced Phototoxicity on the Human Retina during Temporal Approach Eye Surgery" Ophthalmol. 2001, 108(2):381-385; H.F. McDonald and M.J. Harris "Operating microscope-induced retinal phototoxicity during pars plana vitrectomy" Arch. Ophtalmol. 1988 106:521-523; Harris M.D. et al. "Laser eye injuries in military occupations" Avicat.Space Environ. Med. 2003, 74(9):947-952). Damage may also occur upon treatment of ablated surface of corneas after excimer laser phototherapy (Seiji Hayashi et al. "Oxygen free radical damage in the cornea after excimer laser therapy" Br. J. Ophthalmol. 1997, 81:141-144).

Certain corneal disorders are not correctable and may be remedied only by corneal transplant, while others may be corrected by phototherapeutic keratectomy (PTK), i.e., eximer laser surgery, the process of which is also known to cause an inflammatory response, causing corneal hazing or areas of corneal opacification.

The skin around the eyes is also subject to disease and disorders. In particular, rosacea of the eyelids and blepharitis are disorders which can be severe. Ocular rosacea is a common and potentially blinding eye disorder with an uncertain etiology (Stone D.U. and J. Chodosh, 2004 Curr. Opin. Ophthalmol. 15(6):499-502). Blepharitis of the eyes may be Staphylococcal blepharitis, seborrheic blepharitis, mixed forms of these, or the most severe form, ulcerative blepharitis.

Oxidative stress has been implicated in the development or acceleration of numerous ocular diseases or disorders, including AMD and the various retinopathies described above (see, *e.g.*, Ambati et al., 2003, Survey of Ophthalmology 48: 257-293; Berra et al., 2002, Arch. Gerontol. Geriatrics 34: 371-377), as well as uveitis (*e.g.*, Zamir et al., 1999, Free Rad. Biol. Med. 27: 7-15), cataract (*e.g.*, M. Lou, 2003, Prog. Retinal & Eye Res. 22: 657-682), glaucoma (*e.g.*, Babizhayev & Bunin, 2002, Curr. Op. Ophthalmol. 13: 61-67), corneal and conjuctival inflammations. various corneal dystrophies, post-surgical or UV-associated corneal damage (*e.g.*, Cejkova et al., 2001, Histol. Histopathol. 16: 523-533; Kasetsuwan et al., 1999, Arch. Ophthalmol. 117: 649-652), and presbyopia (Moffat et al., 1999, Exp. Eye Res. 69: 663-669). For this reason, agents with anti-oxidative properties have been investigated as potential therapeutic agents for the treatment of such disorders. Many investigations have focused on the biochemical pathways that generate reducing power in cells, for example, glutathione synthesis and cycling. Enzymes, such as superoxide dismutase, that reduce activated oxygen species have also been studied to whether they diminish cellular oxidative stress. Compounds for inhibiting lipid oxidation in cell membranes by direct radical scavenging have also benn considered to be promising therapeutic interventions.

Nitroxides are free radicals that are reducible to their corresponding hydroxylamines. These compounds are of interest because of their radical scavenging properties, mimicking the activity of superoxide dismutase and exerting an anti-inflammatory effect in various animal models of oxidative damage and inflammation. Nilsson *et al.* disclosed, in WO 88/05044, that nitroxides and their corresponding hydroxylamines are useful in prophylaxis and treatment of ischemic cell damage. Paolini *et al.* (U.S. Patent 5,981,548) disclosed N-hydroxylpiperidine compounds and their potential general utility in the treatment of pathologies arising from oxygen radicals and as foodstuff and cosmetic additives. Hsia *et al.* (U.S. Patents 6,458,758, 5,840,701, 5,824,781, 5,817,632, 5,807,831, 5,804,561, 5,767,089, 5,741,893, 5,725,839 and 5,591,710) disclosed the use of stable nitroxides and hydroxylamines (*e.g.*, tempol and its hydroxylamine counterpart, tempol-H), in combination with a variety of biocompatible macromolecules, to alleviate free radical toxicity in blood and blood components. Hahn *et al.* (1998, Int. J. Radiat. Oncol. Biol. Phyics 42: 839-842; 2000, Free Rad. Biol. Med. 28: 953-958) reported on the in vivo radioprotection and effects on blood pressure of the stable free radical nitroxides and certain hydroxylamine counterparts.

In ocular disorders, Zamir *et al.* (1999, *supra*) reported that the nitroxide 4-hydroxy-2,2,6,6,-tetramethylpiperidine-1-N-oxyl (TPL or tempol) reduced the severity of retinal S-antigen-induced experimental autoimmune uveoretinitis (EAU) after systemic injection in a rat model. Reddan et al. (1993, Exp. Eye Res. 56: 543-554) reported an investigation into the use of the nitroxide tempol to protect lens epithelial cells from hydrogen peroxide damage *in vitro.* Mitchell et al. (U.S. Patent 5,462,946) also disclosed use of nitroxides (such as tempol) to protect lens epithelial cells from oxidative damage. Though Mitchell *et al.* also reported that the corresponding hydroxylamine tempol-H afforded no such protection (Mitchell et al., 1991, Arch. Biochem. Biophys. 289: 62-70; Krishna et al., 1991, Cancer Research 51: 6622-6628), Zigler et al. (U.S. Patent 6,001,853) reported to the contrary, disclosing that the hydroxylamine was a better anti-cataractogenic composition than the corresponding nitroxide.

Due to their comparative lack of toxicity, hydroxylamines are preferable to nitroxides as therapeutic agents. However, outside the highly reducing environment of the ocular lens (M. Lou, 2003, *supra*), there has been no report of the use of hydroxylamine compositions for the treatment of diseases or disorders of the eye. Accordingly, the remains a substantial, yet unmet, need for safe, clinically useful, non-surgical treatment for AMD and the numerous other diseases and disorders of the retina and interior of the eye, as well as other ocular disorders.

### SUMMARY OF THE INVENTION

The invention is defined by the claims which are incorporated entirely in the specification by reference.

According to one aspect of the invention, a use is provided for ameliorating, delaying or preventing the development of, or reducing the symptoms of a disease or disorder of the eye of a patient, wherein the diseases or disorder is retinopathy glaucoma and uveitis, cystoid macular edema, as well as certain disorders of the cornea, eyelids and conjunctiva, including but not limited to inflammation, trauma, retinal phototoxicity induced by exposure to operating microscope, laser eye injuries, treatment of ablated surface of corneas after excimer laser phototherapy; presbyopia, irradiation damage, corneal neovascularization and various dystrophies such as Fuch's dystrophy, keratoconus, lattice dystrophy and map-dot-fingerprint dystrophy, blepharitis, rosacea of the eye, as well as dry eye syndrome. In some extraocular embodiments, the compounds and compositions are useful in treating alopecia and preventing/treating damage to rectal tissue due to irradiation therapy. For ocular therapy, the use comprises administering to the eye of the patient a composition comprising an ophthalmologically acceptable carrier or diluent and a hydroxylamine compound in a therapeutically sufficient amount to ameliorate, delay or prevent the development of, or reduce the symptoms of the disease or disorder. For extraocular therapy, the use comprises administering to the affected area of the patient a composition comprising an ophthalmologically acceptable carrier or diluent and a hydroxylamine compound in a therapeutically sufficient amount to ameliorate, delay or prevent the development of, or reduce the symptoms of the disease or disorder.

In one embodiment, the disease or disorder is retinopathy and the retinopathy is classified as choroidal neovascular membrane, macular edema, epi-retinal membrane or macular hole. In another embodiment, the disease or disorder is uveitis and the uveitis is classified as anterior, intermediate, posterior, or diffuse uveitis. In another embodiment the disease or disorder is glaucoma and the glaucoma is classified as high-tension (IOP) or normal-tension glaucoma. In other embodiments, the disease or disorder is of the cornea, eyelid or conjunctiva, particularly as related to oxidative associated with inflammation, trauma, UV irradiation or aging.

In certain embodiments, the compositions contain at least one hydroxylamine compound that is tempol-H. tempo-H or oxano-H. In an exemplary embodiment, the hydroxylamine is tempol-H.

In certain embodiments, the use further comprises administering a reducing agent to the eye of the patient. The reducing agent may be coadministered with the composition or it may be administered separately. The reducing agent is preferably a sulfhydryl compound.

In the foregoing method, the composition may be administered by a variety of routes. Examples include administration by injection, administration from a polymeric disk or wafer (*e.g.*, a contact lens) placed upon the surface of the eye, administration through a cannula or from a device implanted in the patient's body within or in proximity to the eye, or administration from an eye drop, eye wash or eye ointment.

The compositions of the invention may contain more than one compound of the invention. Furthermore, the compositions may contain another compound known in the art for use in the treatment of a particular indication in combination with the compound(s) of the invention. In some embodiments, the compounds of the invention are administered simultaneously. In other embodiments, the compounds of the invention are administered sequentially. Likewise, other compounds known in the art for use in the treatment of the diseases and disorders described herein may be administered with the compound(s) of the invention either simultaneously or sequentially. The invention also provides uses of treatment of diseases and disorders using such combination therapy.

Other features and advantages of the invention will be understood by reference to the detailed description and examples that follow.

### BRIEF DESCRIPTION OF THE DRAWINGS

**Figure 1** shows that various concentrations of Tempol-H protect blue light illuminated A2E-laden RPE cells.
**Figure 2** shows the effect of 1 mM Tempol-H in protection of blue light-illuminated A2E-laden RPE cells.

### DETAILED DESCRIPTION OF ILLUSTRATIVE EMBODIMENTS

The present invention provides uses for the or prevention of a number of ocular diseases or disorders for which current pharmacological therapeutics are sparse or entirely lacking. In particular, uses are provided for the amelioration of diabetic retinopathy and other retinopathies, including but not limited to choroidal neovascular membrane (CNVM), macular edema, epi-retinal membrane (ERM) (macular pucker) and macular hole, as well as glaucoma, uveitis, and disorders of the cornea (and surrounding eyelids and conjunctiva), including but not limited to inflammation, trauma, irradiation damage, corneal neovascularization and various dystrophies such as Fuch's dystrophy, keratoconus, lattice dystrophy and map-dot-fingerprint dystrophy, as well as dry eye syndrome, blepharitis and rosacea of the eye. Uses are also provided to reduce, prevent or ameliorate photooxidative damage to retinal pigment epithelium, which is relevant to development of macular degeneration and for amelioration of irritation and inflammation during laser surgery of the eye, including trabeculectomy treatment for glaucoma and keratectomy for corneal reshaping. The uses comprise administration of a composition comprising an ophthalmologically acceptable carrier or diluent and a hydroxylamine compound in a therapeutically sufficient amount to prevent, retard the development or reduce the symptoms of one or more of the above-listed ophthalmic conditions.

As mentioned, U.S. Patent No. 6,001,853 to Zigler et al*.* disclosed the use of hydroxylamines, preferably combined with reducing agents, for the inhibition of cataract development in the lens of the eye. However, the crystalline lens possesses several unique features to enable it to maintain transparency so that light can be transmitted and focused on the retina. These include (1) a high content of reduced glutathione (GSH), (2) an unusually high protein content (35-50% of its wet weight) and, significantly (3) a variety of antioxidants and oxidation defense enzymes (M. Lou, 2003, *supra*). The unique conditions that exist within the lens may explain why hydroxylamine compounds are found to be effective there for the prevention of cataracts. But because those conditions are not replicated elsewhere in the eye, the efficacy of hydroxylamines as anti-cataractogenics in the ocular lens provides no prediction that compounds of this class will have any efficacy in the amelioration of AMD, retinopathies, inflammatory conditions of any part of the eye, neuropathies, or any other disease or condition outside the lens. Further, the report by Zigler *et al.* that hydroxylamines are anti-cataractogenic within the lens does not address other conditions within the lens, such as presbyopia.

The present inventors have determined that diseases or disorders of the interior and posterior of the eye, and in other tissues outside the lens (*e.g.*, cornea) also can be ameliorated or prevented through the administration of hydroxylamine compounds such as tempol-H. This determination was made through the use of animal models of macular degeneration and other retinal disorders, the protocols of which are set forth in the examples.

Preferred examples of the type of hydroxylamine compounds suitable for use in the present invention are tempol-H ((the hydroxylamine reduced form of the nitroxide 4-hydroxy-2,2,6,6-tetramethylpiperidin-1-yloxy), tempo-H (the hydroxylamine reduced form of the nitroxide 2,2,6,6-tetramethylpiperidin-1-yloxy) and oxano-H (2-ethyl-2,4,4-trimethyloxazolidine, which is the reduced form of oxano, 2-ethyl-2,4,4-trimethyloxazolidin-3-yloxy). Other hydroxylamine compounds suitable for use in the present invention include, but are not limited to, those disclosed by Hahn *et al.* (1998, *supra*; 2000, *supra*), Samuni *et al.* (2001, *supra*); and in U.S. Patent 5,981,548 to Paolini, et al*.* (disclosing certain N-hydroxylpiperidine esters and their use as antioxidants in a number of contexts, none ophthalmologic); U.S. Patent 4,404,302 to Gupta et al. (disclosing the use of certain N-hydroxylamines as light stabilizers in plastics formulations); U.S. Patent 5,462,946 to Mitchell *at al.* (disclosing certain nitroxides deriving from substituted oxazolidines for protection of organisms from oxidative stress); U.S. Patent 3,936,456 to Ramey et al. (disclosing substituted piperazine dione oxyls and hydroxides for the stabilization of polymers); U.S. Patent 4,691,015, to Behrens et al. (describing hydroxylamines derived from hindered amines and the use of certain of them for the stabilization of polyolefins); and the hydroxylamine compounds disclosed in the several aforementioned U.S. patents to Hsia *et al.* Most of the above-referenced compounds have not been known heretofore for administration to the eye. Certainly, none of them has been known for use in the treatment of, macular degeneration, retinopathies, glaucoma or uveitis, or disorders of the cornea, eyelid or conjunctiva.

The uses of the invention involve formulating one or more of the aforementioned (or other suitable) hydroxylamine compounds into compositions for application to the eye of patients in need of therapy. Thus, such compositions are adapted for pharmaceutical use as an injectable agent, or as an eye drop or in contact lenses, inserts or the like, as described in greater detail below. Accordingly, formulation of compound into sterile water containing any desired diluents, salts, pH modifying materials and the like as are known to persons skilled in the pharmaceutical formulations art may be performed in order to achieve a solution compatible with administration to the eye. It may be that injectables, eye drops, inserts, contact lenses, gels and other liquid forms may require somewhat different formulations. All such formulations consistent with direct administration to the eye are comprehended hereby.

The compositions may also have antioxidants in ranges that vary depending on the kind of antioxidant used. The usage also depends on the amount of antioxidant needed to allow at least 2 years shelf-life for the pharmaceutical composition. One or more antioxidants may be included in the formulation. Certain commonly used antioxidants have maximum levels allowed by regulatory authorities. As such, the amount of antioxidant(s) to be administered should be enough to be effective while not causing any untoward effect. Such doses may be adjusted by a physician as needed, within the maximum levels set by regulatory authorities, and is well within the purview of the skilled artisan to determined the proper and effective dose. Reasonable ranges are about 0.01 %: to about 0.15% weight by volume of EDTA, about 0.01% to about 2.0% weight volume of sodium sulfite, and about 0.01% to about 2.0% weight by volume of sodium metabisulfite. One skilled in the art may use a concentration of about 0.1 % weight by volume for each of the above. N-Acetylcysteine may be present in a range of about 0.1% to about 5.0% weight by volume, with about 1% to about 10% of hydroxylamine concentration being preferred. Ascorbic acid or salt may also be present in a range of about 0.1% to about 5.0% weight by volume with about 1% to about 10% weight by volume of hydroxylamine concentration preferred. Other sulfhydryls, if included, may be the same range as for N-acetylcysteine. Other exemplary compounds include mercaptopropionyl glycine, N-acetyl cysteine, β-mercaptoethylamine, glutathione and similar species, although other anti-oxidant agents suitable for ocular administration, *e.g.*, ascorbic acid and its salts or sulfite or sodium metabisulfite may also be employed.

A buffering agent may be used to maintain the pH of eye drop formulations in the range of about 4.0 to about 8.0; so as to minimize irritation of the eye. In certain embodiments, the pH is maintained at about 3.5 to about 6.0, preferably about 4.0 to about 5.5, in order to ensure that most of the hydroxylamine is in its protonated form for highest aqueous solubility. The buffer may be any weak acid and its conjugate base with a pKa of about 4.0 to about 5.5; *e.g.*, acetic acid/sodium acetate; citric acid/sodium citrate. The pKa of the hydroxylamines is about 6.0. For direct intravitreal or intraocular injection, formulations should be at pH 7.2 to 7.5, preferably at pH 7.3-7.4.

The ophthalmologic compositions may also include tonicity agents suitable for administration to the eye. Among those suitable is sodium chloride to make formulations of the present invention approximately isotonic with 0.9% saline solution.

In certain embodiments, the compositions are formulated with viscosity enhancing agents. Exemplary agents are hydroxyethylcellulose, hydroxypropylcellulose, methylcellulose, and polyvinylpyrrolidone. The viscosity agents may be present in the compounds up to about 2.0% weight by volume. It may be preferred that the agents are present in a range from about 0.2% to about 0.5% weight by volume. A preferred range for polyvinylpyrrolidone may be from about 0.1% to about 2.0% weight by volume. One skilled in the art may prefer any range established as acceptable by the Food and Drug Administration.

The compounds used in accordance with the methods of the invention may have co-solvents added if needed. Suitable cosolvents may include glycerin, polyethylene glycol (PEG), polysorbate, propylene glycol, mannitol and polyvinyl alcohol. The presence of the co-solvents may exist in a range of about 0.2% to about 4.0% weight by volume. It may be preferred that mannitol may be formulated in the compounds of the invention in a range of about 0.5% to about 4.0% weight by volume. It may also be preferred that polyvinyl alcohol may be formulated in the compounds of the invention in a range of about 0.1% to about 4.0% weight by volume. One skilled in the art may prefer ranges established as acceptable by the Food and Drug Administration.

Preservatives may be used in the invention within particular ranges. Among those preferred are up to 0.013% weight by volume of benzalkonium chloride, up to 0.013% weight by volume of benzethonium chloride, up to 0.5% weight by volume of chlorobutanol, up to 0.004% weight by volume or phenylmercuric acetate or nitrate, up to 0.0 1 % weight by volume of thimerosal, and from about 0.01% to about 0.2% weight by volume of methyl or propylparabens.

Formulations for injection are preferably designed for single-use administration and do not contain preservatives. Injectable solutions should have isotonicity equivalent to 0.9% sodium chloride solution (osmolality of 290-300 mOsmoles). This may be attained by addition of sodium chloride or other co-solvents as listed above, or excipients such as buffering agents and antioxidants, as listed above. Injectable formulations are sterilized and, in one embodiment, supplied in single-use vials or ampules. In another embodiment, injectable products may be supplied as sterile, freeze-dried solids for reconstitution and subsequent injection.

The tissues of the anterior chamber of the eye are bathed by the aqueous humor, while the retina is under continuous exposure to the vitreous. These fluids/gels exist in a highly reducing redox state because they contains antioxidant compounds and enzymes. Therefore, it may be advantageous to include a reducing agent in the ophthalmologic composition formulated in accordance with the invention, or to dose separately with a reducing agent to maintain the hydroxylamine in its reduced form.

Preferred reducing agents may be N-acetylcysteine, ascorbic acid or a salt form, and sodium sulfite or metabisulfite, with ascorbic acid and/or N-acetylcysteine or glutathione being particularly suitable for injectable solutions. A combination of N-acetylcysteine and sodium ascorbate may be used in various formulations. A metal chelator antioxidant, such as EDTA (ethylenediaminetetraacetic acid) or possibly DTPA (diethylenetriaminepentaacetic acid) may also be added to keep the hydroxylamine in the reduced form in the eye drop formulation.

Compositions utilized in accordance with the methods of the invention may be delivered to the eye of a patient in one or more of several delivery modes known in the art. In a preferred embodiment, the compositions are topically delivered to the eye in eye drops or washes. In another embodiment, the compositions are delivered in a topical ophthalmic ointment, which is particularly useful for treating conditions of the cornea, conjunctiva or surrounding skin, such as dry-eye and blepharitis. In another embodiment, the compositions may be delivered to various locations within the eye *via* periodic subconjunctival or intraocular injection, or by infusion in an irrigating solution such as BSS® or BSS PLUS® (Alcon USA, Fort Worth, TX) or by using pre-formulated solutions of the hydroxylamines in excipients such as BSS® or BSS PLUS®. In one embodiment, the use of the compounds of the invention in vitrectomy may be effective in reducing or preventing the development of vitrectomy-associated cataracts.

Alternatively, the compositions may be applied in other ophthalmologic dosage forms known to those skilled in the art, such as pre-formed or *in situ*-formed gels or liposomes, for example as disclosed in U.S. Patent 5,718,922 to Herrero-Vanrell. A direct injection of drugs into the vitreous body used for treating diseases has been used, in which microspheres or liposomes were used to release drugs slowly (Moritera. T. et al. "Microspheres of biodegradable polymers as a drug-delivery system in the vitreous" Invest. Ophthalmol. Vis. Sci. 1991 32(6): 1785-90).

In another embodiment, the composition may be delivered to or through the lens of an eye in need of treatment *via* a contact lens (*e.g.*, Lidofilcon B, Bausch & Lomb CW79 or DELTACON (Deltafilcon A) or other object temporarily resident upon the surface of the eye. For example, U.S. Pat. No. 6,410,045 describes a contact lens-type drug delivery device comprising a polymeric hydrogel contact lens containing drug substance in a concentration of between 0.05% and 0.25% by weight absorbed in said contact lens which is capable of being delivered into the ocular fluid.

In other embodiments, supports such as a collage corneal shield (*e.g.*, BIO-COR dissolvable, corneal shields, Summit Technology, Watertown, Mass.) can be employed. The compositions can also be administered by infusion into the eyeball, either through a cannula from an osmotic pump (ALZET®, Alza Corp., Palo Alto, Calif.) or by implantation of timed-release capsules (OCCUSENT®) or biodegradable disks (OCULEX®, OCUSERT®) which contain the compositions. These routes of administration have the advantage of providing a continuous supply of the composition to the eye. This may be an advantage for local delivery of the hydroxylamine compounds to the cornea and aqueous humor, for example.

Several other types of delivery systems are available that are particularly suitable for delivering pharmaceutical compositions to the interior or posterior of the eye. For instance, U.S. Patent 6,154,671 to Parel et al. discloses a device for transferring a medicament into the eyeball by iontophoresis. The device utilizes a reservoir for holding the active agent, which contains at least one active surface electrode facing the eye tissue lying at the periphery of the cornea. The reservoir also has a return electrode in contact with the patient's partly closed eyelids. U.S. Patent 5.869,079 to Wong et al. discloses combinations of hydrophilic and hydrophobic entities in a biodegradable sustained release ocular implant. In addition, U.S. Patent 6,375,972 to Guo et al., U.S. Patent 5,902,598 to Chen et al., U.S. Patent 6,331,313 to Wong et al.*,* U.S. Patent 5,707,643 to Ogura et al., U.S. Patent 5,466,233 to Weiner et al. and U.S. Patent 6,251,090 to Avery et al. each describes intraocular implant devices and systems that may be used to deliver pharmaceutical compositions comprising compounds of the present invention.

Many examples of ocular implants for drug delivery are known in the art. For instance, U.S. Patent No. 6,726,918 describes methods for treating inflammation-mediated conditions of the eye comprising: implanting into the vitreous of the eye of an individual a biodegradable implant comprising a steroidal anti-inflammatory agent and a biodegradable polymer, wherein the implant delivers the agent to the vitreous in an amount sufficient to reach a concentration equivalent to at least about 0.05 µg/ml dexamethasone within about 48 hours and maintains a concentration equivalent to at least about 0.03 µg/ml dexamethasone for at least about three weeks.

U.S. Patent No. 6,713,081 describes ocular implant devices for the delivery of a therapeutic agent to an eye in a controlled and sustained manner. Dual mode and single mode drug delivery devices are illustrated and described. Implants suitable for subconjunctival and intravitreal placement are described. The patent also describes fabrication and implementation techniques associated with the ocular implant devices.

U.S. Patent No. 6,251,090 describes an intravitreal medicine delivery device, method and implant device through which a wide variety of beneficial medicines including drugs or other pharmacological agents can be introduced into the vitreous cavity over an extended period of time with only a single initial surgery to implant the device. The device and method minimize the surgical incision needed for implantation and avoid future or repeated invasive surgery or procedures. Additional amounts of the initial medicine can readily be introduced or the medication can be varied or changed, as required. Furthermore, the device and method allow the dosage delivered to the vitreous cavity to be controlled and allows the patient to control the timing of the delivery. The device is constructed so as to filter medicines delivered to the cavity and also avoids damage to or interference with other parts of the eye during implantation or during use.

U.S. Patent No.5,824,072 describes biocompatible ocular implants comprising active agents that are employed for introduction into a suprachoroidal space or an avascular region of an eye for therapeutic purposes. The administration of drugs is controlled and maintained for long periods of time, while ensuring the substantial absence of significant levels outside the site of administration.

U.S. Patent No. 5,773,019 describes a continuous release drug delivery implant which, among other mentioned places, can be mounted either on the outer surface of the eye or within the eye. A drug core is covered by a polymer coating layer that is permeable to the low solubility agent without being release rate limiting.

U.S. Patent No. 5,773,021 describes bioadhesive ophthalmic inserts that are placed in the conjunctival sac. The inserts are prepared by extrusion, thermoforming, or heat compression of a polymeric material matrix and the drug to be delivered. The polymeric matrix comprises a water-soluble biocompatible polymer, such as hydroxyalkyl celluloses, maltodextrins, chitosans, modified starches or polyvinyl alcohols; a water-insoluble biocompatible polymer such as an alkyl cellulose. Where applicable, a bioadhesive polymer such as polyvinyl carboxylic acid type polymers or certain bioadhesive polysaccharides or derivatives thereof may be used. The ophthalmic inserts are characterized therein as intended for the prolonged and controlled release of a medicinal substance.

U.S. Patent Nos. 5,443,505 and 5,766,242 disclose implants comprising active agents for introduction into a suprachoroidal space or an avascular region of the eye, and describe placing microcapsules and plaques comprising hydrocortisone into the pars plana.

U.S. Patent No. 5,378,475 describes a sustained-release implant for insertion into the vitreous of the eye. The implant has a first impermeable coating, such as ethylene vinyl acetate, surrounding most, but not all, of a drug reservoir and a second permeable coating, such as a permeable crosslinked polyvinyl alcohol, disposed over the first coating including the region where the first coating does not cover the drug reservoir, to provide a location through which the drug can diffuse out of the implant.

U.S. Patent No. 5,725,493 describes an ocular implant device for providing drugs to the vitreous cavity over a period of time. The drug reservoir is attached to the outside of the eye with a passageway permitting medicament to enter the vitreous cavity of the eye.

U.S. Patent No. 5,164,188 discloses encapsulated agents for introduction into the suprachoroid of the eye, and describes placing microcapsules and plaques comprising hydrocortisone into the pars plana.

U.S. Patent No. 4,997,652 discloses biodegradable ocular implants comprising microencapsulated drugs, and describes implanting microcapsules comprising hydrocortisone succinate into the posterior segment of the eye.

U.S. Patent No. 4,014,335 describes an ocular drug delivery device placed in the cul-de-sac between the sclera and lower eyelid for administering the drug and acting as a reservoir. The ocular device is characterized therein as administering drug to the eye in a controlled, continuous dosage rate over a prolonged time. To accomplish this, the ocular device comprises a three-layered laminate of polymeric materials holding the drug in a central reservoir region of the laminate. The drug diffuses from the reservoir through at least one of the polymeric layers of the laminate.

U.S. Patent No. 4,300,557 teaches a capsule which can be filled with a pharmaceutical drug to be delivered which serves as an intraocular implant. The capsule is inserted in the vitreous region of the eye by making an incision in the eye, inserting the capsule and closing the incision. The capsule remains in place for a period of time and may be removed by making a second surgical incision into the eye and retrieving the device. The capsule has an attached tube which passes through the surface of the eye and extends outward from the eye useful for the subsequent injection of a drug. While in the vitreous, the device is not anchored and may move about freely.

Further, Zhou *et al.* discloses a multiple-drug implant comprising 5-fluorouridine, triamcinolone, and human recombinant tissue plasminogen activator for intraocular management of proliferative vitreoretinopathy (PVR) (Zhou, T, et al. 1998, "Development of a multiple-drug delivery implant for intraocular management of proliferative vitreoretinopathy" J. Controlled Release 55:281-295).

Compositions in accordance with the methods of the invention are formulated and administered so as to apply a dosage effective for alleviating oxidative stress in the interior and posterior of the eye, and/or inhibiting the of macular degeneration, other retinopathies or uveitis in the eye, among other utilities as discussed herein. In general, it may be preferred that the active amount be from about 0.1% to about 10.0% weight by volume in the formulation. In some embodiments, it is preferable that the active drug concentration be 0.25% to about 10.0% weight by volume. The concentration of the hydroxylamine component will preferably be in the range of about 0.1 µM to about 10 mM in the tissues and fluids. In some embodiments, the range is from 1 µm to 5 mM, in other embodiments the range is about 10 µM to 2.5 mM. In other embodiments, the range is about 50 µM to 1 mM. Most preferably the range of hydroxylamine concentration will be from 1 to 100 µM. The concentration of the reducing agent will be from 1 µM to 5 mM in the tissues and fluids, preferably in the range of 10 µM to 2 mM. The concentrations of the components of the composition are adjusted appropriately to the route of administration, by typical pharmacokinetic and dilution calculations, to achieve such local concentrations.

The uses of this invention can be applied to fields broader than ophthalmology. These areas may include, for example, protection of hair follicles and rectum from radiation damage during radiation therapy for cancer. Other forms of administration, wherein the delivery to the eye is not called for, may include oral tablets, liquids and sprays; intravenous, subcutaneous and intraperitoneal injections; application to the skin as a patch or ointment; enemas, suppositories, or aerosols.

The compositions of the invention may contain more than one compound of the invention. Thus the compositions of the invention contain at least one compound of the invention. In some embodiments, the compounds of the invention are administered simultaneously. In other embodiments, the compounds of the invention are administered sequentially. The methods of the invention include combination therapy.

In some embodiments of the invention, the compound(s) of the invention are administered with another compound known in the art that is useful for treating a disease or disorder that is the target of the compounds of the invention. Thus the composition of the invention may further contain at least one other compound known in the art for treating the disease or disorder to be treated. The other compound(s) known in the art may be administered simultaneously with the compound(s) of the invention, or may be administered sequentially. Similarly, the methods of the invention include using such combination therapy.

For effective treatment of the retinopathies or eye conditions described herein, one skilled in the art may recommend a dosage schedule and dosage amount adequate for the subject being treated. It may be preferred that dosing occur one to four times daily for as long as needed. The dosing may occur less frequently if the compositions are formulated in sustained delivery vehicles, or are delivered *via* implant or intravitreal injection. The dosage schedule may also vary depending on the active drug concentration, which may depend on the hydroxylamine used and on the needs of the patient.

An ophthalmologist or one similarly skilled in the art will have a variety of means to monitor the effectiveness of the dosage scheme and adjust dosages accordingly. For example, effectiveness in the treatment of macular degeneration or other retinopathies may be determined by improvement of visual acuity and evaluation for abnormalities and grading of stereoscopic color fundus photographs. (Age-Related Eye Disease Study Research Group, NEI, NIH, AREDS Report No. 8, 2001, Arch. Ophthalmol. 119: 1417-1436). Effectiveness in the treatment of uveitis may be determined by improvement in visual acuity and vitreous haze and control of inflammation (Foster et al., 2003, Arch. Ophthalmol. 121: 437-40). Following such evaluation, the ophthalmologist may adjust the frequency and/or concentration of the dose, if needed.

The following examples are set forth to describe the invention in greater detail. They are intended to illustrate, not to limit, the invention.

### Example 1: Effect of Tempol-H Treatment on Photochemical Retinal Injury in a Rabbit Model.

The ability of light to produce retinal injury well below the levels capable of producing thermal damage has been termed photochemical retinal injury. The mechanism of action for photochemical retinal injury is believed to be the light induced production of free radicals. The ability of commonly used light sources in clinical ophthalmology to produce photochemical retinal injury is well recognized. The operating microscopes used in ophthalmic surgery have been shown to have the capability of producing retinal photochemical injury. This observation has been utilized to produce a model in the rabbit eye to test the ability of various agents to block photochemical retinal injury. It has been determined that opthalmoscopically visible retinal lesions are detectable after as little as 2.5 minutes of to an operating microscope. The protocol set forth below is utilized to determine whether tempol-H given *via* intravitreal injection has the ability to block the development of an ophthalmoscopically visible photochemical retinal lesion produced by a operating microscope.

*Materials and Methods:* One week prior to treatment, a baseline Fundus photograph and FA is performed on each animal. One day prior to being exposed to the light from a operating microscope, one eye of each animal receives an intravitreal injection of 0.1 cc of BSS® as a control and the fellow eye receives 0.1 cc of tempol-H in a BSS® vehicle. The animals are anesthetized with a IM injection of a 60%-40% mixture of ketamine hydrochloride 100mg/ml and Xylazine 20mg/ml. A speculum is inserted into the eye receiving the injection, and the injection is performed using a 30g needle passed through the sclera just behind the limbus and angled to clear the lens, placing the tip in the mid vitreous cavity. Following injection, the intraocular pressure is monitored using a hand- held Applanation tonometer. If necessary, a paracentesis is performed to return intraocular pressure to normal levels, prior to returning the animals to their.

Forty eyes in 20 pigmented rabbits are exposed to the light from a Zeiss model OpMi 1 operating microscope for various periods of time up to one hour. The rabbits are anesthetized with a IM injection of a 60%-40% mixture of ketamine hydrochloride 100mg/ml and Xylazine 20mg/ml. The rabbits are then positioned on a table; a lid speculum is inserted in the eye to be exposed. The pupil is dilated with tropicamide HCL 1%, and the cornea irrigated with BSS®. The light from the microscope is positioned 20 cm from the animal in a manner so that the light is centered and parallel to the eye. The light filaments are centered in sharp focus on the cornea. Forty-eight hours after exposure, the animals are examined and a repeat Fundus photograph and FA performed. The animals are killed using standard techniques and selected eyes are harvested and stored in chilled glutaldehyde solution, for additional analyses.

### Example 2: Evaluation of Tempol-H Incorporation into Retinal Tissue of Rabbits Following Intravitreous Injection

To assess the efficacy of tempol-H at preventing AMD and other retinal disorders, it must be determined that the drug is incorporated into retinal tissue. The protocol set forth in this example utilizes scintillation technique to establish quantity and duration of tempo-H incorporation into retinal tissue following intravitreous injection in the rabbit. The New Zealand White Rabbit is well characterized in experiments involving intravitreous injection as the large eye of the rabbit provides a suitable template for treatment administration (Hosseini et al., 2003, Lasers Surg. Med. 32: 265-270). Furthermore, the New Zealand White Rabbit has been used extensively in experiments assessing drug uptake into retinal tissue *via* scintillation technique (Ahmed et al., 1987. J. Pharm. Sci. 76: 583-586).

*Materials and Methods:* Twenty-four New Zealand White Rabbit, all male, weighing between 2.5 and 3 kilograms, are used in this protocol. The rabbits are randomized into one of two treatment arms. Initially, rabbits are given baseline ocular exams to ensure that all eyes are free of irritation and infection. Following randomization and baseline exams, rabbits are anesthetized by subcutaneous injection of 100 mg/ml ketamine / 20 mg/ml xylazine. One drop of commercially available 0.5% proparacaine hydrochloride is applied to both eyes. Rabbits are then treated bilaterally by intravitreous injection in accordance with the masked randomization scheme shown below.

### Masked Treatment Arms:

1. Bilateral intravitreous injection with labeled tempol-H. (N=12)
2. Bilateral intravitreous injection with placebo (vehicle). (N=12)

On Day 1,7, 14 and 28, respectively, six rabbits, three from each treatment arm respectively, are sacrificed with a lethal dose of sodium pentobarbital, Immediately following sacrifice, bilateral enucleation is performed and the retinal tissue is removed for scintillation analysis (retinal tissue may be frozen following enucleation to prevent fluid loss). Retinal tissue is ground into a slurry and dissolved in an alkali or quaternary ammonium compound. Scintillation readings are conducted to quantify the amount of tempol-H present in the retinal tissue.

For statistical analysis, data from all rabbits that are dosed with test articles is considered evaluable. Primary and secondary efficacy variables are for statistical significance. Two-sided nonparametric statistical tests are used, and p < 0.05 is considered statistically significant.

### Example 3: Efficacy Tempol-H in Protection Against Photooxidative Processes in the Retinal Pigment Epithelium (RPE)

*Background:* Although the etiology of atrophic age-related macular degeneration (AMD) is not fully understood, it is generally accepted that AMD begins with the death of retinal pigment epithelial cells, the degeneration of photoreceptor cells, and resultant loss of vision, occurring thereafter. There is a growing body of evidence linking the lipofuscin that accumulates in RPE with the death of these cells. For instance, not only are lipofuscin levels highest in RPE cells underlying the macula, the monitoring of RPE lipofuscin by detection of fundus autofluorescence has also shown that areas of RPE atrophy develop at sites of previously increased fluorescence. Studies concerned with examining associations between RPE lipofuscin and RPE cell death have shown that a major constituent of RPE lipofuscin, the bisretinoid fluorophore A2E, can perturb cellular membranes and can mediate blue light damage to RPE. The photoexcitation of A2E leads to the generation, of singlet oxygen and the addition of the latter to carbon-carbon double bonds along the retinoid-derived side arm of A2E such that epoxides are formed. In this way, A2E is converted into a mixture of compounds (A2E-epoxides) bearing epoxides of varying numbers. These highly reactive epoxides have been shown to damage the cell. Ultimately, the photochemical events provoked by the irradiation of A2E in RPE cells initiates cell death by way of a pathwav that involves the participation of cysteine-dependent proteases (caspases) to cleave cellular substates and that is modulated by the mitochondrial protein bcl-2.

The ability of Tempol-H to protect against A2E-mediated blue light damage was examined.

*Materials and Methods:* ARPE-19 cells that have accumulated A2E in culture were exposed to 430 +/- 20 nm light delivered from a halogen source. This wavelength of light is relevant since (i) the excitation maximum of A2E is approximately 430 nm, and (ii) light of this wavelength reaches the RPE *in vivo* while light of wavelengths longer than 400 nm are not absorbed by the cornea and lens. Cell death in blue light-illuminated A2E-laden RPE was compared with and without pre-treatment (24 hours) with Tempol-H. The loss of cell viability was quantified by:
1. A calorimetric microtiter assay based on ability of healthy metabolically active cells to cleave the yellow tetrazolium salt MTT to purple formazan crystals. Cell viability was assessed 24 hours after blue light illumination. Triplicate wells were assayed in each of 2 experiments.
2. Two-color fluorescence assay in which the nuclei of all dead cells appear red due to staining by a membrane-impermeant dye (Dead Red nuclei acid stain, Molecular Probes) with the nuclei of all cells stained blue with DAPI. Cell viability was assessed 8 hours after blue light illumination. Replicates were assayed by counting cells within 5 microscopic fields within the area of illumination in each well and 3 wells were assayed per experiment. Data are based on 3 experiments.

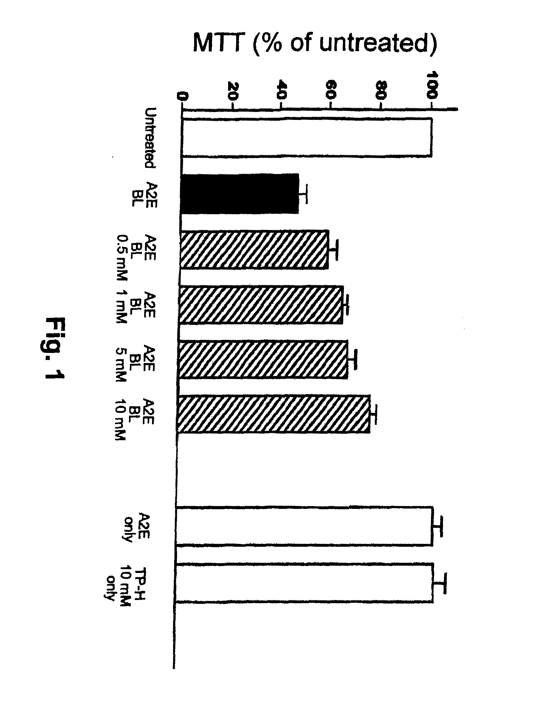

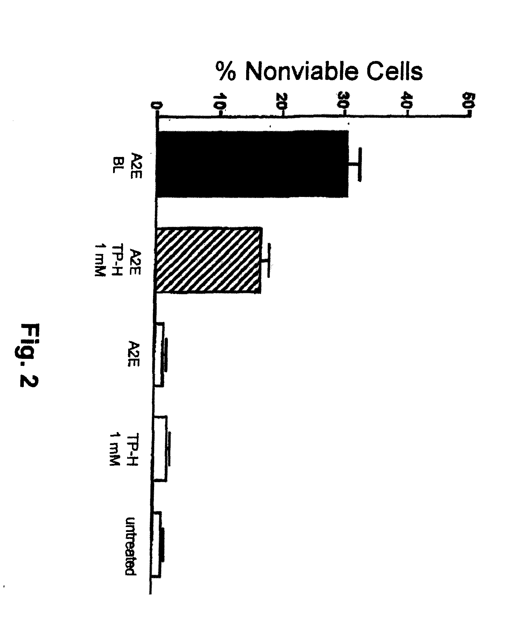

R*esults:* As shown in **Fig. 1** and **Fig. 2**, tempol-H affords a dose-dependent protection against the death of A2E-laden RPE in this model of macular degeneration.

While the present invention has been particularly shown and described with reference to the presently preferred embodiments, it is understood that the invention is not limited to the embodiments specifically disclosed exemplified herein. Numerous changes and modifications may be made to the preferred embodiment of the invention, and such changes and modifications may be made without departing from the scope and spirit of the invention, as set forth in the appended claims.

## Claims

1. Use of at least one hydroxylamine compound for the manufacture of a composition comprising said hydroxylamine compound with an ophtalmologically acceptable carrier or diluent, for ameliorating, delaying or preventing the development of, or reducing the symptoms of a disease or disorder of the eye of a patient, wherein the disease or disorder is retinopathy, corneal diseases, eyelid diseases, presbyopia, glaucoma or uveitis; or for protecting retinal pigment epithelium against photooxidative damage; or for protecting or treating rectal tissue from damage as a result of irradiation.

2. The use of claim 1, wherein the disease or disorder is retinopathy and the retinopathy is classified as choroidal neovascular membrane, macular edema, epi-retinal membrane or macular hole.

3. The use of claim 1 wherein the disease or disorder is uveitis and the uveitis is classified as anterior, intermediate, posterior, or diffuse uveitis.

4. The use of claim 1, wherein the disease or disorder is glaucoma and the glaucoma is classified as high-tension (IOP) or normal-tension glaucoma.

5. The use of claim 1 wherein the disease or disorder is blepharitis or ocular rosacea.

6. The use of any one of claims 1 to 5 wherein said composition comprises said compound in an amount effective to achieve a concentration in the tissues and fluids of about 0.1 µM to about 10 mM, in one or several takings.

7. The use of any one of claims 1 to 5 wherein said composition comprises said compound in an amount effective to achieve a concentration in the tissues and fluids of about 1 µM to about 5 mM, in one or several takings.

8. The use of any one of claims 1 to 5 wherein said composition comprises said compound in an amount effective to achieve a concentration in the tissues and fluids of about 25 µM to about 1 mM, in one or several takings.

9. The use of any one of claims 1 to 5 wherein said composition comprises said compound in an amount effective to achieve a concentration in the tissues and fluids of about 50 µM to about 100 pM, in one or several takings.

10. The use of any one of claims 1 to 9 wherein the disease or disorder is dry eye.

11. The use of any one of claims 1 to 10, wherein the hydroxylamine compound is tempol-H, tempo-H or oxano-H.

12. The use of any one of claims 1 to 13, further comprising using a composition comprising a reducing agent to be applied to the eye of the patient.

13. The use of claim 12, wherein the second composition comprising said reducing agent is formulated to be coadministered with the composition.

14. The use of claim 13, wherein the second composition comprising the reducing agent is formulated as part of the composition.

15. The use of claim 12, wherein the second composition comprising the reducing agent is formulated to be administered separately from the composition.

16. The use of any one of claims 12 to 15, wherein the reducing agent is a sulfhydryl compound.

17. The use of any one of claims 12 to 16, wherein the reducing agent is mercaptopropionyl glycine, N-acetyl cysteine, 3-mercaptoethylamine or glutathione.

18. The use of any one of claims 1 to 17, wherein the composition is formulated to be administered from a polymeric disk or wafer placed upon the surface of the eye.

19. The use of any one of claims 1 to 18, wherein the composition is formulated to be administered through a cannula or from a device implanted in the patient's body within or in proximity to the eye or applied to the surface of the eye by an iontopheresis device.

20. The use of any one of claims 1 to 19, wherein the compositions is formulated to be in an eye drop, eye wash nr eye ointment.
